# EUROPÄISCHE PATENTSCHRIFT

(11) **EP 3 046 479 B1**
(45) Veröffentlichungstag und Bekanntmachung des Hinweises auf die Patenterteilung: **03.10.2018**
(21) Anmeldenummer: 14758107.8
(22) Anmeldetag: 20.08.2014
(51) Int. Cl.: A61B 17/425, A61F 6/04, A61F 6/08

(54) **VORRICHTUNG ZUR ANREICHERUNG VON SPERMATOZOEN**
DEVICE FOR ENRICHING SPERMATOZOA
DISPOSITIF D'ENRICHISSEMENT EN SPERMATOZOÏDES

(30) Priorität: 16.09.2013 DE 102013218528
(43) Veröffentlichungstag der Anmeldung: 27.07.2016
(73) Patentinhaber: BluPink GmbH, 70499 Stuttgart (DE)
(72) Erfinder: PILGRIM, Thorsten, 70839 Gerlingen (DE)
(74) Vertreter: Patentanwälte Ruff, Wilhelm, Beier, Dauster & Partner mbB
(86) Internationale Anmeldenummer: PCT/EP2014/067772
(87) Internationale Veröffentlichungsnummer: WO 2015/036214

(56) Entgegenhaltungen:
- WO-A1-03/086215
- WO-A1-2014/049620
- US-A- 5 640 973
- Rupal I Mehta: "The cause of cessation of viral passage through holes in latex condoms", Journal of Rubber Research, 1. Januar 1998 (1998-01-01), Seiten 1-13, XP055142647, Gefunden im Internet: URL:http://www.fda.gov/downloads/ScienceRe search/SpecialTopics/WomensHealthResearch/ UCM248464.pdf [gefunden am 2014-09-25]

## Beschreibung

Die Erfindung betrifft eine Vorrichtung zum Einführen in einen Vaginalkanal oder zum Aufnehmen eines Penis während eines Koitus zur Anreicherung von Spermatozoen.

Der Wunsch, das Geburtsgeschlecht der Nachkommenschaft beeinflussen zu können ist wahrscheinlich so alt wie die Menschheitsgeschichte selbst. Während über weite Teile der Geschichte insbesondere männliche Nachkommen bevorzugt wurden, scheint das in einer modernen, industrialisierten Welt von deutlich nachrangiger Bedeutung zu sein, oder sich sogar zugunsten von weiblichen Nachkommen zu verschieben.

Auch für ein sogenanntes Ausbalancieren der Familie (Engl. "family balancing"), d.h. zur Herstellung eines Gleichgewichts der Geschlechter innerhalb einer Familie, wünschen sich viele Paare die Möglichkeit, zumindest die Wahrscheinlichkeit für die Zeugung eines Nachkommens eines bestimmten Geschlechts zu erhöhen.

Teilweise ist der Geschlechterwunsch auch medizinisch begründet. Insbesondere im Hinblick auf X-chromosomal-rezessiv vererbbare Erkrankungen besteht der Wunsch, das Geschlecht der Nachkommen bestimmen zu können. Zu dieser Gruppe von bis heute über 500 bekannten Erkrankungen zählen etwa die Hämophilie, die Duchenne-Muskeldystrophie sowie das Lesch-Nyhan-Syndrom. Diese Krankheiten werden durch rezessive Gene auf dem X-Chromosom verursacht, so dass Frauen als Träger dieser Gene damit rechnen müssen, dass ihre Söhne mit einer Wahrscheinlichkeit von 50 % unter der entsprechenden Krankheit leiden werden.

Säugetiersperma enthält in der Regel etwa die gleiche Anzahl von ein Y-Chromosom tragenden Spermatozoen (Y-Spermatozoen) und ein X-Chromosom tragenden Spermatozoen (X-Spermatozoen). Die Befruchtung einer Eizelle durch ein Y-Spermatozoon (auch Y-Spermium oder Y-Spermatozoid genannt) ergibt eine männliche Nachkommenschaft. Die Befruchtung durch ein X-Spermatozoon führt zu einer weiblichen Nachkommenschaft.

Zu dem Thema, wie die Wahrscheinlichkeit, männliche oder weibliche Nachkommen zu zeugen, erhöht werden kann, existiert auf der ganzen Welt eine ganze Reihe an Hausmitteln, die insbesondere den Zeitpunkt des Geschlechtsaktes zwischen Mann und Frau und/oder die Position währenddessen betreffen.

Zu nennen in diesem Zusammenhang sind beispielsweise die sog. STORCH-Parameter, wobei sich STORCH aus den Anfangsbuchstaben von fünf Parametern zusammensetzt, die angeblich das Geschlecht der Nachkommenschaft beeinflussen können:
- Stellung während des Geschlechtsakts
- Timing: Zeitpunkt des Geschlechtsakts im Zusammenhang mit den Mondphasen
- Orgasmus-Zeitpunkt von Frau oder Mann
- Richtige(r) Ernährung / Säure-Basen-Haushalt
- Chinesischer Empfängniskalender
- Hitze: Temperaturunterschiede des männlichen Hodens

Zwar gibt es bezüglich der genannten Parameter, insbesondere in Bezug auf den chinesischen Empfängniskalender, einige erfolgversprechende Statistiken, allerdings ist die entsprechende Anwendung der Verfahren meist mit einem erheblichen zeitlichen Aufwand verbunden. Zudem sind die Anleitungen für das Verfahren meist mit erheblichem Interpretationsspielraum ausgestattet.

Im Rahmen der künstlichen Befruchtung existieren weitere Möglichkeiten und in verschiedenen Ländern ist eine Geschlechterwahl bei der künstlichen Befruchtung erlaubt. In Bezug auf die Behandlung einer Probe für die künstliche Befruchtung sind beispielsweise aus der WO 02/052244 A1 verschiedene Verfahren bekannt, die eine Trennung von X- und Y-Spermatozoen gewährleisten sollen.

Aus der Veröffentlichung Rupal I Mehta: "The cause of cessation of viral passage through holes in latex condoms" (in Journal of Rubber Research, 1 January 1998, Seiten 1 - 13) sind Untersuchungen zu einem Durchtritt von Viren durch eine mit Löchern versehenen Kondomwandung bekannt. Zu diesem Zweck werden Löcher eingebracht, deren Durchmesser zwischen 1,7 und 28,5 µm liegen.

US 5,640,973 beschreibt eine Gestaltung eines Kondoms, durch welches Viren und andere Krankheitserreger zurückgehalten werden.

Aus dem nachveröffentlichten Dokument WO 2014/049620 A1 ist eine Gestaltung eines Kondoms bekannt, durch welche ein Samenfluss positiv in eine gewünschte Richtung gelenkt werden soll, um so die Wahrscheinlichkeit einer Empfängnis zu erhöhen.

Aus dem Dokument WO 03/086251 A1 ist eine ähnlich einem Kondom gestaltete Vorrichtung mit einer porösen, labyrinthartigen Struktur bekannt, durch welche Spermatozoen entsprechend ihrer Viabilität gefiltert werden. Die Vorrichtung wird nach Gebrauch von einem Penis abgezogen und verbleibt dann für mehrere Stunden im Körper der Frau.

Die Erfindung stellt sich die Aufgabe, eine Vorrichtung bereitzustellen, die eine Anreicherung von X- und/oder Y-Chromosom tragenden Spermatozoen ermöglicht, dabei allerdings weder hohe apparative und finanzielle Anforderungen stellt, noch für den Anwender besonders umständlich in der Handhabung ist.

Diese Aufgabe wird erfindungsgemäß gelöst durch eine Vorrichtung umfassend eine selektiv permeable Trennwand mit den Merkmalen der unabhängigen Ansprüche 1 und 4 sowie durch ein Kit mit den Merkmalen des Anspruchs 10. Bevorzugte Ausführungsformen der Vorrichtung und des Kits sind Gegenstand der abhängigen Ansprüche 2 bis 3, 5 bis 9 bzw. 11. Der Wortlaut sämtlicher Ansprüche wird hiermit durch Bezugnahme zum Inhalt der Beschreibung gemacht.

Gemäß einem ersten Aspekt wird eine Vorrichtung in Form eines Pessars oder eines Diaphragmas zum Einführen in den Vaginalkanal einer Benutzerin oder in Form eines Kondoms zum Aufnehmen eines Penis während eines Koitus geschaffen, umfassend eine selektiv permeable Trennwand zur Anreicherung von Y-Chromosom tragenden Spermatozoen nach der Trennwandpassage, wobei die selektiv permeable Trennwand Poren aufweist oder als Sieb oder Gewebe mit Maschen ausgebildet ist, und wobei die Größe der Poren oder der Maschen der selektiv permeablen Trennwand an die Größe der Y-Chromosom tragenden Spermatozoen angepasst ist und eine nominale Porengröße in einem Bereich zwischen ca. 3,800 µm und ca. 4,900 µm und/oder eine Porengröße D90 in einem Bereich zwischen ca. 3,800 µm und ca. 4,900 µm liegt bzw. die Maschen eine Maschenweite in einem Bereich zwischen ca. 3,800 µm und ca. 4,900 µm aufweisen, sodass die selektiv permeable Trennwand eine erhöhte Durchlässigkeit für Y-Chromosom tragende Spermatozoen aufweist.

Als selektiv permeabel zur Anreicherung von Y-Chromosom tragenden Spermatozoen nach der Trennwandpassage wird im Zusammenhang mit der Anmeldung eine Trennwand bezeichnet, die eine erhöhte Durchlässigkeit für Y-Chromosom tragende Spermatozoen aufweist, während X-Chromosom tragende Spermatozoen zurückgehalten werden. Die selektiv permeable Trennwand dient damit der Anreicherung von Y-Chromosom tragenden Spermatozoen stromabwärts der Trennwand.

Die Vorrichtung ist derart gestaltet, dass sie in den Vaginalkanal einer Benutzerin eingeführt werden kann oder während eines Koitus einen Penis aufnehmen kann. Die Vorrichtung ist in anderen Worten ähnlich einem bekannten Barriere-Verhütungsmittel, beispielsweise einem Pessar oder einem Kondom, gestaltet und eine Handhabung erfolgt in entsprechender Art und Weise. Als Pessar werden im Zusammenhang mit der Anmeldung allgemein Produkte bezeichnet, welche in die Vagina eingelegt oder auf den Muttermund aufgesetzt werden. Eine besondere Ausgestaltung eines Pessars ist das Diaphragma.

Die selektiv permeable Trennwand dient vorzugsweise zum Modifizieren eines Ejakulats auf dem Weg zum Muttermund. Die Modifizierung besteht darin, dass das Ejakulat oder zumindest ein Teil des Ejakulats, welcher die selektive permeable Trennwand passiert, nach einer Passage der Trennwand mehr, vorzugsweise ausschließlich, Y-Spermatozoen aufweist, während X-Spermatozoen an der Trennwand zurückgehalten werden.

Wie eingangs bereits erwähnt, enthält Säugetiersperma grundsätzlich etwa die gleiche Anzahl von X- und Y-Spermatozoen. Durch ein mit Hilfe der selektiv permeablen Trennwand modifiziertes Ejakulat lässt sich die Wahrscheinlichkeit erhöhen, männliche (Anreicherung von Y-Spermatozoen nach der Trennwandpassage) Nachkommen zu erzielen.

In anderen Worten ist gemäß der Erfindung die Größe der Poren oder Maschen der selektiv permeablen Trennwand an die Größe der Y-Spermatozoen angepasst, so dass diese die Trennwand passieren können. Auf diese Weise wird auf der Filtratseite eine Anreicherung von Y-Spermatozoen und auf der Unfiltratseite eine Anreicherung von X-Spermatozoen erzielt. Diese zumindest teilweise oder vollständig separierten X- und Y-Spermatozoen können dann direkt zur Befruchtung herangezogen oder - insbesondere bei Verwendung einer über einen Penis gezogenen Vorrichtung - zur späteren Verwendung eingefroren werden.

Die Trennwirkung einer entsprechenden selektiv permeablen Trennwand beruht auf der überraschenden Erkenntnis, dass X- und Y-Spermatozoen ausschließlich aufgrund ihrer Größe zumindest teilweise separiert werden können. Die erfindungsgemäße Trennwand fungiert dabei als eine Art Filter, welcher in Abhängigkeit von der gewählten Porengröße oder Maschenweite die X-Chromosom tragenden Spermatozoen zumindest teilweise, vorzugsweise vollständig, zurückhält (Unfiltratseite) bzw. die Y-Chromosom tragenden Spermatozoen passieren lässt (Filtratseite).

Zwischen X- und Y-Spermatozoen existiert ein im Mikrometerbereich liegender, messbarer Größenunterschied. Demnach hat ein Y-Spermatozoon einen mittleren Kopfumfang von 14,73 µm mit einer Standardabweichung von 1,07 µm. Ein X-Spermatozoon weist dagegen einen mittleren Kopfumfang von 15,26 µm mit einer Standardabweichung von 1,17 µm auf. Somit weist das X-Spermatozoon einen im Mittel um etwa 530 nm größeren Kopfumfang auf. Dieser Unterschied ist im Sinne statistischer Auswerteverfahren höchst signifikant, d.h. die Irrtumswahrscheinlichkeit p ist kleiner als 1 ‰ (p < 0,001).

Mit besonderem Vorteil ist die erfindungsgemäße selektiv permeable Trennwand nicht nur auf eine Anwendung bei menschlichem Sperma bzw. menschlichen Spermatozoen beschränkt, sondern kann bei jeder Art von Säugetiersperma angewendet werden, insbesondere bei Existenz eines Größenunterschieds zwischen X-Chromosom und Y-Chromosom tragenden Spermatozoen.

In vorteilhaften Ausgestaltungen weist die selektiv permeable Trennwand Poren mit einer nominalen Porengröße in einem Bereich zwischen ca. 3,800 µm und ca. 4,500 µm, insbesondere in einem Bereich zwischen ca. 3,900 µm und ca. 4,000 µm auf. Die Poren sind in einer Ausgestaltung aufgrund einer Porosität eines verwendeten porösen Materials gebildet. In anderen Ausgestaltungen werden Poren in ein porenfreies Material eingebracht, insbesondere durch Laserschnitt, Laserbohren, Ätzen oder dergleichen. Die Poren sind in einer Ausgestaltung irregulär geformt. Als Porengröße wird dabei ein Durchmesser eines die Pore einhüllenden Kreises bezeichnet. Poröse Materialien weisen in der Regel Poren unterschiedlicher Porengröße auf. Eine Verteilung der Porengrößen eines Materials wird als Porengrößenverteilung bezeichnet. Als nominale Porengröße im Sinne der Anmeldung wird das Maximum in der Porengrößenverteilung bezeichnet. Bei einer (hypothetischen) kreisförmigen Pore mit einer nominalen Porengröße von 4,900 µm ist ein Umfang der Porenöffnung etwa 15,394 µm.

Erfindungsgemäß ist es in diesem Zusammenhang und im Zusammenhang mit allen folgenden Angaben nominaler Porengrößen bevorzugt, dass zwischen 50 und 100 %, besonders bevorzugt 60 und 99 %, insbesondere 70 und 95 %, der gesamten Poren der Trennwand die angegebene nominale Porengröße aufweisen oder diese zumindest unterschreiten.

Insbesondere weist die Trennwand vorzugsweise Poren mit einer Porengröße D90 in einem Bereich zwischen ca. 3,800 µm und ca. 4,500 µm, insbesondere in einem Bereich zwischen ca. 3,900 µm und ca. 4,000 µm, auf. Als Porengröße D90 wird im Zusammenhang mit der Anmeldung diejenige Porengröße bezeichnet, bei der 90 % des Porenvolumens des Materials eine Porengröße kleiner als die Porengröße D90 aufweisen.

Die Porengrößenverteilung eines Materials kann Quecksilberporosometrie und/oder Gasadsorption bestimmt werden. Diese Methoden sind dem Fachmann prinzipiell bekannt und beispielsweise in den einschlägigen Normen ISO 15901-1 EN, ISO 15901-2 EN, ISO 15901-3 EN sowie DIN 66133, DIN 66134 und DIN 66135 beschrieben. Alternativ erfolgt eine Bestimmung mittels Rasterelektronenmikroskopie, Permeabilitätsmessungen (Gel-Permeations-Chromatographie) und Blasen-Punkt-Test.

In einer anderen Ausgestaltung ist die Trennwand als Gewebe oder Sieb mit Maschen gestaltet, wobei die Maschen eine Maschenweite in einem Bereich zwischen ca. 3,800 µm und ca. 4,900 µm, vorzugsweise in einem Bereich zwischen ca. 3,800 µm und ca. 4,500 µm, insbesondere in einem Bereich zwischen ca. 3,900 µm und ca. 4,000 µm, aufweisen. Als Maschenweite wird dabei eine Länge der Diagonale einer rechteckförmigen Masche bezeichnet. Eine Bestimmung erfolgt beispielsweise mittels Rasterelektronenmikroskopie. Vorzugsweise weist eine erfindungsgemäße Trennwand eine statistische Porenanzahl pro Flächeneinheit zwischen 10000 mm⁻² und 50000 mm⁻², vorzugsweise 15000 mm⁻² und 40000 mm⁻², insbesondere 20000 mm⁻² und 30000 mm⁻², auf.

Experimente mit einer Spermaprobe haben gezeigt, dass bei einer nominalen Porengröße von 4,773 µm, d.h. bei einer (hypothetischen) Pore mit kreisförmigen Querschnitt und einem Umfang der Porenöffnung von etwa 14,995 µm, nach der Passage der selektiv permeablen Trennwand 59,8 % der Y-Spermatozoen und 41,2% der X-Spermatozoen im verbleibenden Probenvolumen auf der Filtratseite vorliegen. Somit hat sich zwar die gesamte Spermienanzahl in etwa halbiert, allerdings entspricht das erhaltene Verhältnis von 59,8 % Y- Spermatozoen zu 41,2 % X-Spermatozoen einer um etwa 23 % höheren Wahrscheinlichkeit für eine Jungengeburt, wenn man von einer idealen Ausgangszusammensetzung der Spermaprobe von 50,0 % Y- Spermatozoen und 50,0 % X-Spermatozoen und einer damit einhergehenden 50 %-igen Wahrscheinlichkeit für eine Jungengeburt ausgeht.

Unter Berücksichtigung der Tatsache, dass das männliche Ejakulat durchschnittlich etwa 5 x 10⁸ X- und Y-Spermatozoen im Verhältnis 1 zu 1 enthält, würden nach der beschriebenen Passage einer erfindungsgemäßen Trennwand mit einer nominalen Porengröße von 4,773 µm noch immer ca. 2,5 x 10⁸ Spermatozoen für eine Befruchtung zur Verfügung stehen.

Bei der Passage einer Spermaprobe durch eine erfindungsgemäße Trennwand mit einer nominalen Porengröße von nur 3,937 µm, d.h. bei einer (hypothetischen) Pore mit kreisförmigen Querschnitt mit einem Umfang der Porenöffnung von etwa 12,368 µm, konnten auf der Filtratseite 1,37 % Y-Spermatozoen und 0,68 % X-Spermatozoen erhalten werden, was bei anfänglich durchschnittlich 5 x 10⁸ Spermatozoen etwa noch 1 % der ursprünglich in der Probe enthaltenen Spermatozoen entspricht. Legt man bezüglich der Ausgangszusammensetzung der Spermaprobe dieselben Annahmen wie oben zugrunde, ergibt sich mit dem erhaltenen Verhältnis von 1,37 % Y- zu 0,68 % X-Spermatozoen eine um etwa 51 % höhere Wahrscheinlichkeit für eine Jungengeburt bei einer für eine Befruchtung zur Verfügung stehenden Spermatozoenanzahl von etwa 1 x 10⁷.

Aufgrund der Durchlässigkeit für Y-Spermatozoen, welche die selektiv permeable Trennwand mit den oben genannten bevorzugten Porengrößen oder Maschenweiten aufweist, lässt sich also die Wahrscheinlichkeit für eine Jungengeburt vorzugsweise um mehr als 15 %, besonders bevorzugt 25 %, insbesondere 50 %, erhöhen, wenn eine nach der Passage der Trennwand erhaltene mit Y-Spermatozoen angereicherte Probe für eine Befruchtung herangezogen wird.

Für eine Fertigung der Trennwand sind verschiedene biokompatible Materialien denkbar, welche vorzugsweise keine Anhaftung oder Adhäsion von Spermatozoen begünstigt. Da ein Ejakulat in der Regel zu einem erheblichen Teil auch proteinartige Stoffe enthält, weist das Material vorzugsweise proteinrepellierende Eigenschaften auf. So lässt sich auch ein ungewolltes Verstopfen der Poren oder Maschen zumindest teilweise vermeiden.

Die Wahl eines geeigneten Materials richtet sich insbesondere nach der Form der Vorrichtung und wie diese verwendet werden soll. Es ist auch denkbar, dass die Trennwand teilweise starr, beispielsweise aus einem keramischen Material, ausgebildet ist, wobei die Vorrichtung vorzugsweise zusätzliche Bereiche aufweist, welche einen Tragekomfort erhöhen.

Dem Fachmann sind geeignete Materialien grundsätzlich bekannt. Auf dem freien Markt gibt es eine Vielzahl an Filtermaterialien, insbesondere für den Bereich der Biowissenschaften, die sich grundsätzlich zur Anfertigung einer erfindungsgemäßen selektiv permeablen Trennwand eignen. Der Vorteil bei der Verwendung solcher Materialien liegt darin, dass diese in der Regel bereits medizinische Zulassungsverfahren durchlaufen haben und als unbedenklich und/oder biokompatibel eingestuft sind.

Vorzugsweise besteht die selektiv permeable Trennwand aus einem Polymer ausgewählt aus der Gruppe mit Kautschuk, Polylaktid, Cellulose, Celluloseacetat, Cellulosenitrat, Polyethylen, Polypropylen, Polyurethan, Polyisopren, Polytetrafluorethylen, Polyvinylchlorid, Polyamid, Polycarbonat, Polyvinylidenfluorid, Polyethersulfon, Polysiloxane und Kombinationen daraus ausgebildet.

Gemäß einer weiteren Ausführungsform ist die selektiv permeable Trennwand zumindest einseitig mit einer Beschichtung, insbesondere einer basische Beschichtung, versehen.

Die basische Beschichtung ist dabei vorzugsweise auf der Filtratseite der Trennwand aufgebracht, also auf der Seite, die zunächst nicht mit dem Ejakulat in Kontakt tritt. Männliches Sperma weist grundsätzlich einen schwach basischen pH-Wert auf (7,2 - 7,8). Mittels einer basischen Beschichtung auf der Filtratseite der Trennwand lässt sich mit besonderem Vorteil die Wanderungstendenz der Spermatozoen auf der Unfiltratseite durch die Poren in Richtung der Filtratseite erhöhen, wenn die Filtration gegen einen Säuregradienten durchgeführt wird. Ein Säuregradient entsteht beispielsweise, wenn in der unmittelbaren Umgebung der Filtratseite der Trennwand ein saures Milieu vorherrscht, während auf der Unfiltratseite, bedingt durch die basische Spermaprobe, ein basisches Milieu vorliegt. Die basische Beschichtung auf der Filtratseite führt in diesem Fall in unmittelbarer Nähe zur Filtratseite zu einem pH-Wert-Ausgleich, so dass die Wanderungsgeschwindigkeit der Spermatozoen in Richtung des sauren Milieus erhöht wird. Die Beschichtung ist dabei in einer Ausgestaltung derart gewählt, dass die Trennwand eine erhöhte Durchlässigkeit für X-Chromosom tragende Spermatozoen aufweist.

Denkbar ist ferner eine Ausführungsform, bei welcher die Beschichtung auf der Filtratseite mit chemischen Lockstoffen für Spermatozoen versehen ist (Chemotaxis). Derartige Lockstoffe sind bekannt. Beispielsweise kann es sich um dieselben Lockstoffe handeln, die von einer Eizelle während des Befruchtungsvorgangs sezerniert werden, um den Spermatozoen den Weg zu weisen (zyklische Nukleotide, cAMP oder cGMP).

Außerdem kann die Trennwand zumindest einseitig mit Bakteriziden, Deodorants oder Gleitmitteln angereichert, imprägniert oder ausgerüstet sein. Weiterhin kann sie mit verschiedenen pharmakologischen Verbindungen wie Hormonen und Derivaten angereichert, imprägniert oder ausgerüstet sein. Fachkundigen Personen sind Methoden der Verbindung mit Medikamenten, Hormonen oder sonstigen pharmakologischen Verbindungen grundsätzlich bekannt.

Gemäß einer besonders bevorzugten Ausführungsform ist die selektiv permeable Trennwand als ein Bestandteil eines Kondoms, eines Diaphragmas oder eines Pessars ausgebildet.

Zur Herstellung eines derartigen Kondoms, Pessars oder Diaphragmas liegt die selektive Trennwand in vorteilhaften Ausgestaltungen zunächst folienförmig oder membranartig in der für ein Kondom bzw. Diaphragma üblichen Stärke und/oder Form vor. Als Materialien bieten sich die üblicherweise für Kondome (beispielsweise Polyisopren, Polyethylen, Polyurethan) und Diaphragmen (beispielsweise Silikon, Polyamid) verwendeten Materialen an. Im Rahmen der Herstellung kann dann ein Teilbereich des Kondoms, insbesondere im Bereich des Spermareservoirs, oder ein Teilbereich des Diaphragmas, insbesondere im Bereich des Flächenmittelpunkts, durch die selektiv permeable Trennwand ersetzt werden. Die Verbindung der Materialen kann beispielsweise durch Verkleben oder Verschweißen erfolgen.

Alternativ ist die selektiv permeable Trennwand als Kondom, Pessar oder Diaphragma geformt. Gemäß dieser Ausführungsform werden vorzugsweise sowohl das Kondom als auch das Diaphragma in ihrer Gesamtheit aus einem auch als Trennwand fungierenden Material gefertigt. Ein in der Regel umständliches Verschweißen oder Verkleben kann so entfallen. Dabei sind in einer Ausgestaltung verstärkte Bereiche, Einlagen oder dergleichen vorgesehen, welche beispielsweise als Spannring für ein Diaphragma dienen. Liegt die selektiv permeable Trennwand in Form eines Kondoms vor, kann es weiterhin bevorzugt sein, dass die Außenseite des Kondoms (entspricht der Filtratseite) mit einer basischen Beschichtung versehen ist. Im weiblichen Vaginalbereich herrscht grundsätzlich ein saures Milieu, so dass die basische Beschichtung wie oben beschrieben lokal einen Ausgleich des pH-Werts bewirkt und so die Migration von im Kondom befindlichen Spermatozoen in Richtung Filtratseite fördert.

Liegt die selektiv permeable Trennwand in Form eines Pessars oder Diaphragmas vor, ist es insbesondere vorgesehen, dass die, nach der Platzierung des Pessars oder Diaphragmas, dem Muttermund zugewandte Seite (entspricht der Filtratseite) mit einer basischen Beschichtung versehen ist. Dadurch lässt sich die Migrationstendenz der Spermatozoen durch die selektiv permeable Trennwand in Richtung des Muttermunds erhöhen.

Ein weiterer Gegenstand der Erfindung ist ein Kit zur Anreicherung von X-Chromosom oder Y-Chromosom tragenden Spermatozoen, umfassend eine Vorrichtung mit einer selektiv permeablen Trennwand gemäß den obigen Ausführungen sowie zumindest ein Gleitmittel.

Vorzugsweise umfasst das Kit eine selektiv permeable Trennwand in Form eines Kondoms oder eines Diaphragmas und/oder als Bestandteil eines Kondoms oder eines Diaphragmas, wobei die einzelnen Bestandteile des Kits bevorzugt räumlich getrennt voneinander und insbesondere steril verpackt sind.

In einer weiteren Ausführungsform des erfindungsgemäßen Kits umfasst das Kit zusätzlich oder anstelle des Gleitmittels ein Mittel zur Erzeugung einer basischen Beschichtung auf der selektiv permeablen Trennwand. Das Mittel kann vorzugsweise auch die Funktion eines Gleitmittels ausüben, so dass das Vorliegen eines separaten Gleitmittels nicht erforderlich ist.

Gemäß einer bevorzugten Ausführungsform umfasst das Kit eine selektiv permeable Trennwand zur Verwendung mit einer Vorrichtung in Form eines Kondoms und/oder in Form eines Diaphragmas sowie ein Gleitmittel und ein Mittel zur Erzeugung einer basischen Beschichtung wie es oben beschrieben wurde.

Weitere Vorteile der Erfindung ergeben sich aus den Unteransprüchen und aus der nachfolgenden Beschreibung von Ausführungsbeispielen der Erfindung, die in den Zeichnungen schematisch dargestellt sind. Für gleiche oder ähnliche Bauteile werden in den Zeichnungen einheitliche Bezugszeichen verwendet. Als Teil eines Ausführungsbeispiels beschriebene oder dargestellte Merkmale können ebenso in einem anderen Ausführungsbeispiel verwendet werden, um eine weitere Ausführungsform der Erfindung zu erhalten.

In den Zeichnungen zeigen:
- **Figur 1**: eine perspektivische Darstellung einer Ausführungsform einer erfindungsgemäßen Vorrichtung in Form eines Diaphragmas;
- **Figur 2**: einen Querschnitt des Diaphragmas aus Figur 1;
- **Figur 3**: einen Querschnitt eines Diaphragmas ähnlich Figur 1;
- **Figur 4**: einen Querschnitt eines Diaphragmas ähnlich Figur 1 mit einer austauschbar aufgenommenen Trennwand und
- **Figur 5**: eine Seitenansicht einer Ausführungsform einer erfindungsgemäßen Vorrichtung in Form eines Kondoms.

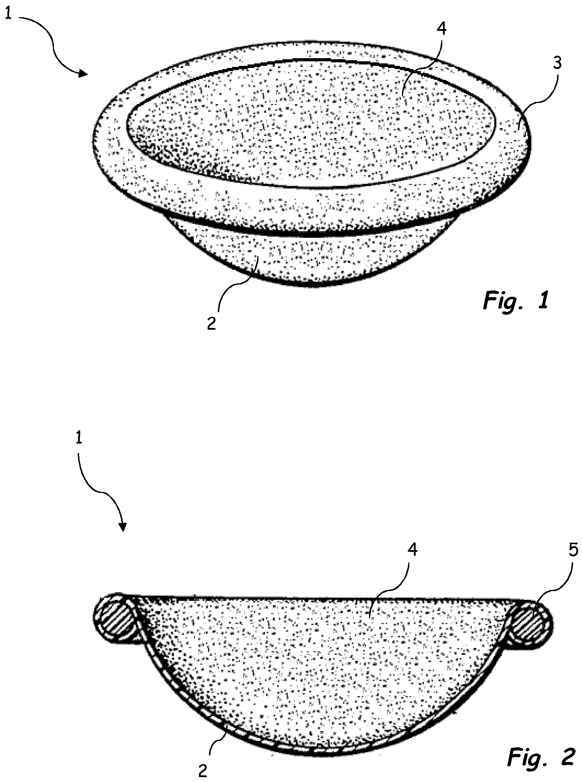

Figur 1 und 2 zeigen eine als kuppelförmiges Diaphragma gestaltete Vorrichtung 1 umfassend eine selektiv permeable Trennwand 2 in einer perspektivischen Darstellung bzw. einem Querschnitt.

Die Trennwand 2 ist als elastische Membran, beispielsweise aus Silikon oder Polyamid gefertigt. Das Diaphragma umfasst weiter einen formstabilen elastischen Ring 3, welcher zum Einsetzen des Diaphragmas in einer Vagina dient und durch welchen gewährleistet wird, dass die Form des Diaphragmas auch nach dem Einbringen in den Körper erhalten bleibt

Die selektiv permeable Trennwand 2 weist schematisch und nicht maßstäblich dargestellte Poren 4 mit einer nominalen Porengröße von 4,773 µm auf. Aufgrund der Wahl der Porengröße weist die selektiv permeable Trennwand eine erhöhte Durchlässigkeit für Y-Chromosom tragende Spermatozoen auf.

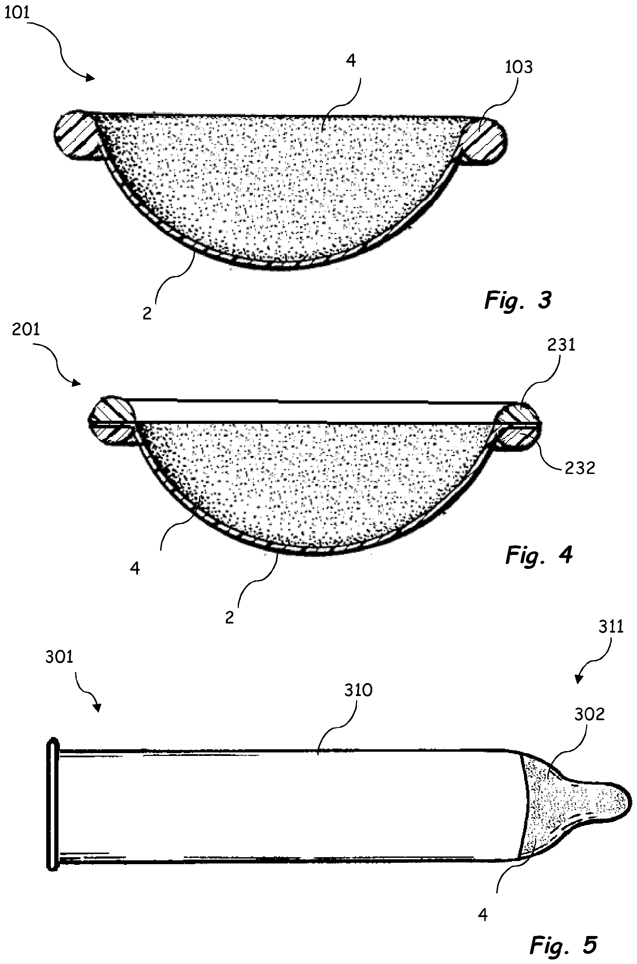

Figur 3 zeigt eine alternative Ausgestaltung einer als kuppelförmiges Diaphragma gestaltete Vorrichtung 101 umfassend eine selektiv permeable Trennwand 2 in einem Querschnitt. Die Gestaltung ist ähnlich den Figuren 1 und 2 und für gleiche Ausgestaltungen werden einheitliche Bezugszeichen verwendet. Im Unterschied zu dem Ausführungsbeispiel gemäß den Figuren 1 und 2 ist das Diaphragma gemäß Figur 3 als integrales Element aus einem Material gefertigt, wobei ein Teilbereich 103 als elastisches Ringelement fungiert.

Figur 4 zeigt eine weitere alternative Ausgestaltung einer als kuppelförmiges Diaphragma gestaltete Vorrichtung 201 umfassend eine selektiv permeable Trennwand 2 in einem Querschnitt. Auch die Gestaltung gemäß Figur 3 ist ähnlich den Figuren 1 und 2 und für gleiche Ausgestaltungen werden einheitliche Bezugszeichen verwendet. Im Unterschied zu dem Ausführungsbeispiel gemäß den Figuren 1 und 2 umfasst die Vorrichtung 201 einen zweiteiligen Ring. Der Ring weist zwei miteinander verbindbaren Ringelemente 231, 232 auf, zwischen welchen eine selektiv permeable Trennwand 2 austauschbar aufgenommen ist. Der Ring, insbesondere dessen Größe und/oder Elastizität, ist dabei individuell an eine Nutzerin anpassbar. Die selektiv permeable Trennwand 2 kann dabei derart gestaltet sein, dass sie für verschiedene Ringgestaltungen verwendbar ist.

Figur 5 zeigt eine Seitenansicht einer als Kondom gestalteten erfindungsgemäßen Vorrichtung 301 mit einer selektiv permeablen Membran 302. Das dargestellte Kondom weist einen röhrenförmigen Bereich 310 und ein Spermareservoir 311 auf, wobei der Bereich des Spermareservoir 311 durch eine selektiv permeable Trennwand 302 gebildet ist. Die Verbindung der Trennwand 301 mit dem röhrenförmigen Bereich erfolgt vorzugsweise durch Verkleben oder Verschweißen. Dabei ist für das Spermareservoir 311 ein anderes Material verwendbar, als für den röhrenförmigen Bereich.

## Patentansprüche

1. Vorrichtung in Form eines Pessars oder eines Diaphragmas zum Einführen in einen Vaginalkanal oder in Form eines Kondoms zum Aufnehmen eines Penis während eines Koitus, umfassend eine selektiv permeable Trennwand (2, 302) zur Anreicherung von Y-Chromosom tragenden Spermatozoen nach der Trennwandpassage, wobei die selektiv permeable Trennwand (2, 302) Poren aufweist, wobei die Größe der Poren der selektiv permeablen Trennwand an die Größe der Y-Chromosom tragenden Spermatozoen angepasst ist und eine nominale Porengröße in einem Bereich zwischen ca. 3,800 µm und ca. 4,900 µm und/oder eine Porengröße D90 in einem Bereich zwischen ca. 3,800 µm und ca. 4,900 µm liegt, sodass die selektiv permeable Trennwand (2, 302) eine erhöhte Durchlässigkeit für Y-Chromosom tragende Spermatozoen aufweist.

2. Vorrichtung nach Anspruch 1, **dadurch gekennzeichnet, dass** selektiv permeable Trennwand (2, 302) Poren (4) mit einer nominalen Porengröße in einem Bereich zwischen ca. 3,800 µm und ca. 4,500 µm aufweist.

3. Vorrichtung nach Anspruch 1 oder 2, **dadurch gekennzeichnet, dass** die selektiv permeable Trennwand (2, 302) Poren (4) mit einer Porengröße D90 in einem Bereich zwischen ca. 3,800 µm und ca. 4,500 µm aufweist.

4. Vorrichtung in Form eines Pessars oder eines Diaphragmas zum Einführen in einen Vaginalkanal oder in Form eines Kondoms zum Aufnehmen eines Penis während eines Koitus, umfassend eine selektiv permeable Trennwand (2, 302) zur Anreicherung von Y-Chromosom tragenden Spermatozoen nach der Trennwandpassage, wobei die selektiv permeable Trennwand (2, 302) als Sieb oder Gewebe mit Maschen ausgebildet ist, wobei die Größe der Maschen der selektiv permeablen Trennwand an die Größe der Y-Chromosom tragenden Spermatozoen angepasst ist und die Maschen eine Maschenweite in einem Bereich zwischen ca. 3,800 µm und ca. 4,900 µm aufweisen, sodass die selektiv permeable Trennwand (2, 302) eine erhöhte Durchlässigkeit für Y-Chromosom tragende Spermatozoen aufweist.

5. Vorrichtung nach Anspruch 4, **dadurch gekennzeichnet, dass** die Maschen eine Maschenweite in einem Bereich zwischen ca. 3,800 µm und ca. 4,500 µm aufweisen.

6. Vorrichtung nach einem der Ansprüche 1 bis 3, **dadurch gekennzeichnet, dass** die selektiv permeable Trennwand (2, 302) eine statistische Porenanzahl pro Flächeneinheit zwischen 10000 mm⁻² und 50000 mm⁻² aufweist.

7. Vorrichtung nach 6, **dadurch gekennzeichnet, dass** die selektiv permeable Trennwand (2, 302) eine statistische Porenanzahl pro Flächeneinheit zwischen 15000 mm⁻² und 40000 mm⁻² aufweist.

8. Vorrichtung nach einem der vorhergehenden Ansprüche, **dadurch gekennzeichnet, dass** die selektiv permeable Trennwand (2, 302) zumindest teilweise aus einem Polymer ausgewählt aus der Gruppe mit Kautschuk, Polylactid, Cellulose, Celluloseacetat, Cellulosenitrat, Polyethylen, Polypropylen, Polyurethan, Polyisopren, Polytetrafluorethylen, Polyvinylchlorid, Polyamid, Polycarbonat, Polyvinylidenfluorid, Polyethersulfon, Polysiloxane und Kombinationen daraus ausgebildet ist.

9. Vorrichtung nach einem der vorhergehenden Ansprüche, **dadurch gekennzeichnet, dass** die selektiv permeable Trennwand (2, 302) zumindest einseitig mit einer basischen Beschichtung versehen ist.

10. Kit zur Anreicherung von X-Chromosom oder Y-Chromosom tragenden Spermatozoen, umfassend eine Vorrichtung (1, 101, 201, 301) mit einer selektiv permeable Trennwand (2, 302) nach einem der Ansprüche 1 bis 9 und ein Gleitmittel.

11. Kit nach Anspruch 10, **dadurch gekennzeichnet, dass** das Kit ferner oder anstelle des Gleitmittels ein Mittel zur Erzeugung einer basischen Beschichtung auf der Trennwand (2, 302) umfasst.

## Claims

1. Device in the form of a pessary or a diaphragm for insertion into a vaginal passage or in the form of a condom for accommodation of a penis during a coitus, comprising a selectively permeable separating wall (2, 302) for the enrichment of Y-chromosome-bearing spermatozoa after passing the separating wall, wherein the selectively permeable separating wall (2, 302) has pores, wherein the size of the pores of the selectively permeable separating wall is adapted to the size of the Y-chromosome-bearing spermatozoa and a nominal pore size is in a range between approx. 3,800 µm and approx. 4,900 µm and/or a pore size D90 is in a range between approx. 3,800 µm and approx. 4,900 µm so that the selectively permeable separating wall (2, 302) shows an increased permeability for Y-chromosome-bearing spermatozoa.

2. Device according to claim 1, **characterized in that** the selectively permeable separating wall (2, 302) has pores (4) with a nominal pore size in a range between approx. 3,800 µm and approx. 4,500 µm.

3. Device according to claim 1 or 2, **characterized in that** the selectively permeable separating wall (2, 302) has pores (4) with a pore size D90 in a range between approx. 3,800 µm and approx. 4,500 µm.

4. Device in the form of a pessary or a diaphragm for insertion into a vaginal passage or in the form of a condom for accommodation of a penis during a coitus, comprising a selectively permeable separating wall (2, 302) for the enrichment of Y-chromosome-bearing spermatozoa after passing the separating wall, wherein the selectively permeable separating wall (2, 302) is designed as sieve or fabric with meshes, wherein the size of the meshes of the selectively permeable separating wall is adapted to the size of the Y-chromosome-bearing spermatozoa and the meshes have a mesh opening in a range between approx. 3,800 µm and approx. 4,900 µm so that the selectively permeable separating wall (2, 302) has an increased permeability for Y-chromosome-bearing spermatozoa.

5. Device according to claim 4, **characterized in that** the meshes have a mesh opening in a range between approx. 3,800 µm and approx. 4,500 µm.

6. Device according to any of the claims 1 to 3, **characterized in that** the selectively permeable separating wall (2, 302) has a statistic number of pores per unit area between 10000 mm⁻² and 50000 mm⁻².

7. Device according to claim 6, **characterized in that** the selectively permeable separating wall (2, 302) has a statistic number of pores per unit area between 15000 mm⁻² and 40000 mm⁻².

8. Device according to any of the preceding claims, **characterized in that** the selectively permeable separating wall (2, 302) at least partially is made of a polymer selected from the group comprising rubber, polylactide, cellulose, cellulose acetate, cellulose nitrate, polyethylene, polypropylene, polyurethane, polyisoprene, polytetrafluoroethylene, polyvinyl chloride, polyamide, polycarbonate, polyvinylidene fluoride, polyethersulfone, polysiloxane and combinations thereof.

9. Device according to any of the preceding claims, **characterized in that** the selectively permeable separating wall (2, 302) is at least on one side provided with a basic coating.

10. Kit for the enrichment of X-chromosome or Y-chromosome bearing spermatozoa, comprising a device (1, 101, 201, 301) with a selectively permeable separating wall (2, 302) according to any of the claims 1 to 9 and a lubricant.

11. Kit according to claim 10, **characterized in that** the kit further or instead of the lubricant comprises a substance for generating a basic coating on the separating wall (2, 302).

## Revendications

1. Dispositif sous la forme d'un pessaire ou d'un diaphragme destiné à être introduit dans un canal vaginal ou sous la forme d'un préservatif destiné à recevoir un pénis pendant un coït, comprenant une paroi de séparation à perméabilité sélective (2, 302) destinée à la concentration de spermatozoïdes portant un chromosome Y après le passage de la paroi de séparation, la paroi de séparation à perméabilité sélective (2, 302) comprenant des pores, la taille des pores de la paroi de séparation à perméabilité sélective étant adaptée à la taille des spermatozoïdes portant un chromosome Y, et une taille de pore nominale se situant dans une plage comprise entre environ 3 800 µm et environ 4 900 µm et/ou une taille de pore D90 se situant dans une plage comprise entre environ 3 800 µm et environ 4 900 µm, de telle sorte que la paroi de séparation à perméabilité sélective (2, 302) présente une perméabilité augmentée pour les spermatozoïdes portant un chromosome Y.

2. Dispositif selon la revendication 1, **caractérisé en ce que** la paroi de séparation à perméabilité sélective (2, 302) comprend des pores (4) ayant une taille de pore nominale dans une plage comprise entre environ 3 800 µm et environ 4 500 µm.

3. Dispositif selon la revendication 1 ou 2, **caractérisé en ce que** la paroi de séparation à perméabilité sélective (2, 302) comprend des pores (4) ayant une taille de pore D90 dans une plage comprise entre environ 3 800 µm et environ 4 500 µm.

4. Dispositif sous la forme d'un pessaire ou d'un diaphragme destiné à être introduit dans un canal vaginal ou sous la forme d'un préservatif destiné à recevoir un pénis pendant un coït, comprenant une paroi de séparation à perméabilité sélective (2, 302) destinée à la concentration de spermatozoïdes portant un chromosome Y après le passage de la paroi de séparation, la paroi de séparation à perméabilité sélective (2, 302) étant configurée sous la forme d'un tamis ou d'un tissu à mailles, la taille des mailles de la paroi de séparation à perméabilité sélective étant adaptée à la taille des spermatozoïdes portant un chromosome Y, et les mailles présentant une largeur de maille dans une plage comprise entre environ 3 800 µm et environ 4 900 µm, de telle sorte que la paroi de séparation à perméabilité sélective (2, 302) présente une perméabilité augmentée pour les spermatozoïdes portant un chromosome Y.

5. Dispositif selon la revendication 4, **caractérisé en ce que** les mailles présentent une taille de mailles dans une plage comprise entre environ 3 800 µm et environ 4 500 µm.

6. Dispositif selon l'une quelconque des revendications 1 à 3, **caractérisé en ce que** la paroi de séparation à perméabilité sélective (2, 302) présente un nombre de pores statistique par unité de surface compris entre 10 000 mm⁻² et 50 000 mm⁻².

7. Dispositif selon la revendication 6, **caractérisé en ce que** la paroi de séparation à perméabilité sélective (2, 302) présente un nombre de pores statistique par unité de surface compris entre 15 000 mm⁻² et 40 000 mm⁻².

8. Dispositif selon l'une quelconque des revendications précédentes, **caractérisé en ce que** la paroi de séparation à perméabilité sélective (2, 302) est formée au moins partiellement en un polymère choisi dans le groupe constitué par le caoutchouc, le polylactide, la cellulose, l'acétate de cellulose, le nitrate de cellulose, le polyéthylène, le polypropylène, le polyuréthane, le polyisoprène, le polytétrafluoroéthylène, le polychlorure de vinyle, le polyamide, le polycarbonate, le polyfluorure de vinylidène, la polyéthersulfone, les polysiloxanes et leurs combinaisons.

9. Dispositif selon l'une quelconque des revendications précédentes, **caractérisé en ce que** la paroi de séparation à perméabilité sélective (2, 302) est munie d'un revêtement basique sur au moins un côté.

10. Kit pour la concentration de spermatozoïdes portant un chromosome X ou un chromosome Y, comprenant un dispositif (1, 101, 201, 301) comprenant une paroi de séparation à perméabilité sélective (2, 302) selon l'une quelconque des revendications 1 à 9 et un agent lubrifiant.

11. Kit selon la revendication 10, **caractérisé en ce que** le kit comprend en plus ou à la place de l'agent lubrifiant un agent pour la formation d'un revêtement basique sur la paroi de séparation (2, 302).
